# EUROPEAN PATENT APPLICATION

(11) **EP 2 796 558 A1**
(43) Date of publication of application: **29.10.2014**
(21) Application number: 13164966.7
(22) Date of filing: 23.04.2013
(51) Int. Cl.: C12N 15/82

(54) **Improved gene targeting and nucleic acid carrier molecule, in particular for use in plants**

(71) Applicant: Rheinische Friedrich-Wilhelms-Universität Bonn, 53113 Bonn (DE)
(72) Inventor: Hommelsheim, Carl, 50374 Erfstadt (DE); Frantzeskakis, Lamprinos, 40237 Düsseldorf (DE); Ülker, Bekir, 53115 Bonn (DE)
(74) Representative: Krauss, Jan

(57) **Abstract**

The present invention relates to a nucleic acid carrier molecule, comprising the general formula M-S₁-L-W-S₂, wherein M is a first polypeptide specifically binding to a donor nucleic acid sequence to be transferred into an organelle of a cell, W is a second polypeptide specifically binding to a target nucleic acid sequence, wherein said target nucleic acid sequence is located in an organelle of a cell, L is missing or is linking group allowing M and W flexibility and semi-independence, and S₁ and S₂ independently of each other are missing or are a signal peptide sequence, and can be fused to M and W proteins either N-or C-terminally, wherein said donor nucleic acid sequence is brought into close proximity with said target nucleic acid sequence when both nucleic acid sequences are bound to said carrier molecule. The present invention furthermore relates to methods for recombinantly transforming a nucleic acid into an organelle in a cell, preferably a plant cell, employing said nucleic acid carrier molecule.

## Description

The present invention relates to a nucleic acid carrier molecule, comprising the general formula M-S₁-L-W-S₂, wherein M is a first polypeptide specifically binding to a donor nucleic acid sequence to be transferred into an organelle of a cell, W is a second polypeptide specifically binding to a target nucleic acid sequence, wherein said target nucleic acid sequence is located in an organelle of a cell, L is missing or is linking group allowing M and W flexibility and semi-independence, and S₁ and S₂ independently of each other are missing or are a signal peptide sequence, and can be fused to M and W proteins either N-or C-terminally, wherein said donor nucleic acid sequence is brought into close proximity with said target nucleic acid sequence when both nucleic acid sequences are bound to said carrier molecule. The present invention furthermore relates to methods for recombinantly transforming a nucleic acid into an organelle in a cell, preferably a plant cell, employing said nucleic acid carrier molecule.

### Background of the invention

Gene targeting (also, replacement strategy based on homologous recombination) is a genetic technique that uses homologous recombination to change an endogenous gene. The method can be used to delete a gene, remove exons, add a gene, and introduce point mutations. Gene targeting can be permanent or conditional. Conditions can be a specific time during development / life of the organism or limitation to a specific tissue, for example. Gene targeting requires the creation of a specific vector for each gene of interest.

Gene targeting has long been a major goal for researchers interested in the production of transgenic plants with stable and predictable patterns of gene expression. Unfortunately gene targeting in plants has been extremely difficult. To successfully achieve gene targeting requires homology between the gene integrated in the genomic DNA (target) and the new gene designed to repair or replace it (donor).

The frequency of gene targeting can be significantly enhanced through the use of engineered endonucleases such as zinc finger nucleases, engineered homing endonucleases, and nucleases based on engineered TAL effectors (Miller et al., 2011).

To date, this method has been applied to a number of species including Arabidopsis (Lloyd et al., 2005), tobacco (Wright et al., 2005; Cai et al., 2009; Townsend et al., 2009), and corn (Shukla et al., 2009).

TALEs (transcription-activator like effectors) are proteins injected into plants by plant pathogenic bacteria of the genus *Xanthomonas* (Kay and Bonas, 2009). TALEs are able to modulate expression of plant genes necessary to promote microbe-infection (Kay et al., 2007). A central repeat domain within the protein structure mediates DNA recognition and each repeat binds specifically to a single nucleotide (Boch et al., 2009; Moscou and Bogdanove, 2009). Since the rearreagment of these repeats is possible, they can be assembled in a user-specific manner in order to recognize a target DNA sequence (Cermak et al., 2011; Miller et al., 2011). The binding domain of TALEs, in conjunction with functional domains coming from other proteins such as nucleases (e.g FoKI), methylases, repressors, etc., can be used as tools for genome editing in eukaryotic cells.

Based on the target sequence as chosen, TAL-effectors can target any chosen region in the chromosome, and then control the gene expression using an activator- or repressor unit. Currently, TAL-effectors are mainly used together with a nuclease-, activator- and/or repressor function (for examples, see Morbitzer et al., 2010; Romer et al., 2010; Li et al., 2012; Mahfouz et al., 2012).

WO 2011/072246 and US 2011-145940 describe a method for modifying the genetic material of a cell, comprising providing a cell containing a target DNA sequence; and introducing a transcription activator-like (TAL) effector -DNA modifying enzyme into the cell, the TAL effector-DNA modifying enzyme comprising: (i) a DNA modifying enzyme domain that can modify double stranded DNA, and (ii) a TAL effector domain comprising a plurality of TAL effector repeat sequences that, in combination, bind to a specific nucleotide sequence in the target DNA sequence, such that the TAL effector-DNA modifying enzyme modifies the target DNA within or adjacent to the specific nucleotide sequence in the cell or progeny thereof, Novel DNA-binding proteins and uses thereof.

WO 2010/079430 describes a method for producing a polypeptide that selectively recognizes at least one base pair in a target DNA sequence, the method comprising synthesizing a polypeptide comprising a repeat domain, wherein the repeat domain comprises at least one repeat unit derived from a transcription activator-like (TAL) effector.

WO 03/080809 discloses a method of generating a genetically modified cell, comprising providing a primary cell containing an endogenous chromosomal target DNA sequence in which it is desired to have homologous recombination occur; providing a zinc finger endonuclease comprising an endonuclease domain that cuts DNA, and a zinc finger domain comprising a plurality of zinc fingers that bind to a specific nucleotide sequence within said endogenous chromosomal target DNA.

WO 2009/042186 discloses methods and compositions for genomic editing of one or more genes in zebrafish, using fusion proteins comprising a zinc finger protein and a cleavage domain or cleavage half-domain. Polynucleotides encoding said fusion proteins are also provided, as are cells comprising said polynucleotides and fusion proteins.

*Agrobacterium tumefaciens* is the causative agent of crown gall disease in plants. As a consequence of natural gene transfer from the pathogen to the plant cells, infected plant tissue produces tumors and synthesizes nutrients which only Agrobacteria is able to use. This natural gene transfer mechanism has been successfully exploited by researchers in generating transgenic plants and gave rise to the *Agrobacterium*-mediated transformation method. *Agrobacterium*-mediated transformation is the most preferred method used for the genetic engineering of plant cells for basic research and crop improvements for agriculture (Tzfira and Citovsky, 2006). The method is relatively simple and leads to insertion of 1-3 copies of transgenes in average (Alonso et al., 2003). Direct transformation methods such as microprojectile bombardment in contrast, requires much more skilled handling and generates transgenic lines with undesired complex transgene insertions (Ülker et al., 1999). Although most plant species are the natural hosts for *Agrobacterium,* this microorganism can also transform a wide range of other eukaryotic species including fungi, yeast, sea urchin and even human cells (Lacroix et al., 2006). This genetic transformation is achieved by transporting a single-stranded copy (T-strand) of the bacterial transferred DNA region (T-DNA region) from the tumor-inducing (Ti) plasmid into the plant cell nucleus, followed by integration into the host genome (Gelvin, 2009; Pitzschke and Hirt, 2010). In *Agrobacterium,* the T-region is delimited by two 25-bp direct repeats, the T-DNA borders, that are cleaved by the bacterial VirD2 endonuclease to produce a transferable T-strand molecule. Any DNA placed between the T-DNA borders were shown to be transported into the plant cell nucleus. Molecular events that occur in the bacterial cell prior to T-DNA transfer are reasonably well understood (McCullen and Binns, 2006; Pitzschke and Hirt, 2010).

The mechanism of nuclear import of T-DNA requires bacterial virulence (Vir) proteins, in particular the protein VirD2. Two 25-bp imperfect direct repeats, termed border sequences, define the T-DNA. This single-stranded T-DNA-VirD2 complex is transferred to the plant cell and subsequently enters the nucleus, and the T-DNA is finally integrated into the plant cell genome. Proteins imported into the nucleus generally contain motifs composed of one or two stretches of basic amino acids. These motifs are termed nuclear localization signals (NLSs) and are recognized by the nuclear import machinery. VirD2 contains two NLSs, and the C-terminal NLS of VirD2 has been shown to be required for efficient transfer of the bacterial T-DNA to the plant nucleus (Ziemienowicz et al., 2001).

Together with VirD1, VirD2 forms a relaxosome at the RB of the T-region, which initiates the formation of the T- strand. VirD1 and VirD2 are both required for recognition of the border repeat sequences flanking the T-strand but it is the relaxase domain of the VirD2 protein which actually cleaves the LB and RB sequence on one of the DNA strands, enabling the release of the T-strand (Jayaswal et al., 1987; Lessl and Lanka, 1994; Scheiffele et al., 1995). VirD2 remains covalently attached to the 5' end of the T-strand through an N-terminal tyrosine residue (Tyr29) (Durrenberger et al., 1989; Vogel and Das, 1992; Scheiffele et al., 1995).

Although there has been some progress in DNA targeting of plants, still the situation is far from being satisfying [see, for example, Urnov et al. (Urnov et al., 2010)].

One of the most important requirements for efficient genome editing in any organism is delivering nucleic acids to desired genomic DNA regions and keeping them there until editing by host repair machinery. Although, it is currently possible to deliver proteins and peptides to specific organelles and regions of DNA, it is not possible to deliver nucleic acids to defined genomic DNA regions. Up to now it was not possible to send nucleotide sequences to such a close proximity to influence where and how they should integrate.

Fauser et al. (Fauser et al., 2012) speculate about an enhancement of gene targeting frequencies if the target and the donor locus are located on the same chromosome. The authors further generated three independent transgenic lines and sequentially crossed them to induce recombination after cleavage by a transgenic endonucleases, and therefore used a cumbersome system that takes long and also requires the generation and crossing of transgenic plants.

It is therefore an object of the present invention, to overcome the above shortcomings, and to provide suitable and convenient tools and methods for efficient gene targeting, in particular in plants.

According to a first aspect of the present invention, this object is solved by a nucleic acid carrier molecule, comprising the general formula

M-S₁-L-W-S₂,

wherein M is a first polypeptide specifically binding to a donor nucleic acid sequence to be transferred into an organelle of a cell, W is a second polypeptide specifically binding to a target nucleic acid sequence, wherein said target nucleic acid sequence is located in an organelle of a cell, L is missing or is linking group allowing M and W flexibility and semi-independence, and S₁ and S₂ independently of each other are missing or are a signal peptide sequence, and can be fused to M and W proteins either N-or C-terminally, wherein said donor nucleic acid sequence is brought into close proximity with said target nucleic acid sequence when both nucleic acid sequences are bound to said carrier molecule.

It was surprisingly found that when the target and donor DNA sequences that are specifically bound by the polypeptides (or domains of a fusion construct) M and W are brought into close proximity, and are thus immediately available for the cellular machinery, e.g. for recombination and/or repair, an effective and specific gene targeting, also designated as "genome editing" can be achieved.

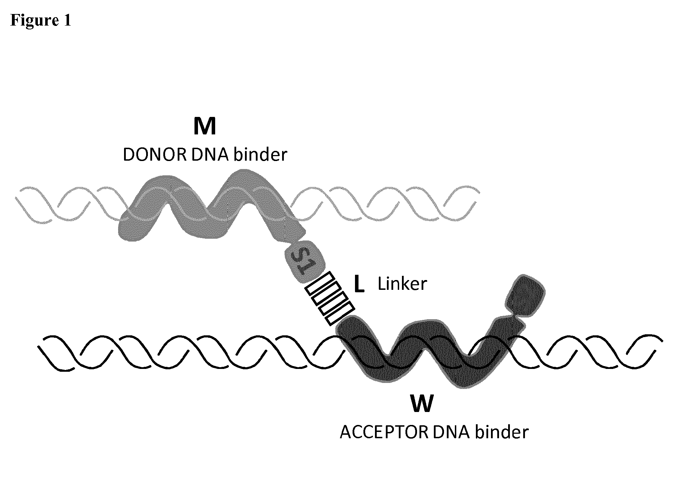

The technology underlying the present invention is based on engineered polypeptides comprising (or consisting of) two polypeptides or domains M and W, optionally connected with a (poly)peptide linker (L), each binding to the same or different donor and target nucleotide sequence, respectively (Figure 1). The present invention relies on the fact that the MLW construct has the ability to search and specifically bind to the donor (bound by M) and the target sequences (bound by W), and to bring them together (into close proximity) in an organelle (nucleus, chloroplast or mitochondrium), where the target DNA is located (Figure 1).

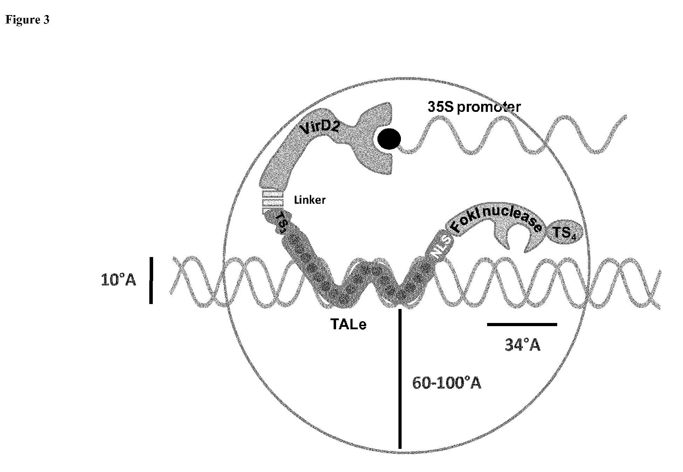

In the context of the present invention, "proximity" or "close proximity" shall be understood as a distance between two molecules (in this case the donor and target sequences in the organelle) that they are immediately available for the recombination or repair mechanisms in the organelle. Due to the flexibility and free movement of the M polypeptide, the donor sequence can be positioned as close as 10 angstrom to the target nucleotides. The maximum distance would be 200 angstrom to the target. Preferred is thus a proximity of between 10-200 angstrom. Preferred is a proximity between the two molecules of less than about 100 angstrom, more preferably of less than about 50 angstrom, and most preferred of less than about 20 angstrom. Still more preferred are between 10 and 20 angstrom (see Figure 3)

Consequently, in a preferred MLW molecule, L is missing or is linking group, such as, for example, selected from a polypeptide linker or an organic linker group which is covalently connecting M and W, wherein said linker provides for a proximity as described above. The linker can be a branched or non-branched molecule/group, and can comprise additional functions, such as, for example, provide attachment points for additional functional groups, such as marker groups (labels) and/or chemical groups for attaching the molecule to a substrate (e.g. chelating groups or other coupling groups). Preferred is a group L that is less than about 30 amino acids in length, more preferably less than about 15 amino acids in length, and most preferred of less than about 10 amino acids in length. The chemical structure of the linker is usually chosen so as to not interfere with the binding properties of the groups M and/or W, and thus allows for a flexibility of the M and W groups, which is semi-independent, i.e. the linker is sufficient in that neither M inhibits the steric movement of W, nor W inhibits the steric movement of M.

In some combinations of M and W, the binding as well as other desired tasks for example activation/repression of transcription, DNA cleavage carried out by the M and/or W polypeptides may be hindered if M and W are directly fused without any spacer between them. Therefore, in a preferred embodiment of the present invention, a - preferably short (see above) - linker peptide is added, acting as a spacer that does not hinder M and W functions to be performed independently of each other. Similarly, in order for the donor DNA to be better used as template in, for example, targeted gene editing or homologous recombination, this DNA and its ends should be as close as possible to the target especially to the DNA cleavage site. Since the W polypeptide anchors the MLW protein-DNA complex to the target DNA, for example, in a nucleus or target organelle, the M polypeptide should have some flexibility to swing the donor DNA, so that this DNA can be better used for recombination or insertion/deletions by the host DNA repair complexes. Therefore, the addition of a linker allows the M polypeptide to move more flexibly.

One of the major applications for this discovery is targeted engineering of eukaryotic and prokaryotic genomes. Targeted engineering in genomes requires the specific recognition of a DNA sequence in the host genome and efficient delivery of the modified DNA sequence not only to the host cell but even more importantly bringing this DNA very close proximity of the target sequence. The present technology satisfies both these requirements.

The MLW molecule according to the present invention is highly instrumental in altering host genomic DNA by mutations, deletions or inserting new DNA sequences. Preferably, MLW molecules help in significantly improving homologous recombination in plants which until now is very difficult to achieve. In addition, human diseases and disorders linked to genetic defects could also be efficiently repaired using the MLW molecules. Most importantly, viral sequences integrating into host genomes, like devastating HIV virus, could be permanently deactivated by insertion or deletion of its DNA sequences, and viral host receptors could be mutated to block virus infections. Thus, preferred is a nucleic acid carrier molecule according to the present invention, wherein the target nucleic acid sequence is selected from viral sequences, mutated sequences, and sequences that are organelle-specific.

Advantageously, the present invention allows for precise genome editing in both prokaryotes and eukaryotes, and the efficient generation of genetically modified organisms, in particular in plants. The present invention also improves the transformability and efficiency of transformation in organisms, such as plants, improves safety of genetically engineered organisms by reducing superfluous and off-target DNA integration in undesired locations in host genomes. As mentioned above, human, animal and plant diseases and disorders linked to genetic defects can also be efficiently repaired using MLW proteins, if the underlying genetic origin is known.

Other preferred aspects include the precise delivery of miRNA, RNAi, and antisense RNAs to desired locations in genomes for efficient gene therapy in humans, animals and plants, the delivery of single strand or double strand oligos to desired locations in genomes for genome editing, the delivery of fluorescently or radioactively labeled nucleotides to desired locations in genomes for marking, detection and manipulations, stem cell genome editing and subsequent correction of faulty alleles of human, animal or plant genes causing abnormalities and diseases.

Furthermore, the disadvantages of viral DNA delivery systems in humans are eliminated by using MLW constructs, the expression of a particular gene can be modulated by replacing or editing its promoter region. Also, the expression of, for example, hundreds of genes in a pathway could be regulated by changing the promoter region of the particular transcription factors regulating these genes in the host. Similarly, such transcription factors could be introduced as additional transgene under the desired promoter and terminator.

The MLW constructs according to the present invention could also be used without any additional DNA to engineer host genomes. In this embodiment, the researcher could rely on the presence of complementing pieces of DNAs located in minimum two different positions in the genome. For example, using the construct according to the present invention a disease causing dominant allele in a heterozygous organism can be replaced by the normal allele in the same genome without any additional DNA delivery.

Finally, organelles such as mitochondria and chloroplasts which are difficult to transform and hence to edit their genome can be edited with the MLW technology according to the present invention by simply adding organelle targeting signal peptides to the W (and/or M) polypeptide.

Preferred is a nucleic acid carrier molecule according to the present invention, wherein M is selected from the group consisting of a TAL effector (TALe) polypeptide, a zinc finger polypeptide, a VirD2-like polypeptide, a VirE2-like polypeptide, an RNA binding polypeptide, and a transcription factor polypeptide, wherein said polypeptide specifically binds to said donor nucleic acid.

Further preferred is a nucleic acid carrier molecule according to the present invention, wherein W is selected from the group of a TAL effector (TALe) polypeptide, a zinc finger polypeptide, a VirD2-like polypeptide, a VirE2-like polypeptide, an RNA binding polypeptide, and a transcription factor polypeptide, wherein said polypeptide specifically binds to said target nucleic acid sequence that is located in said organelle.

Thus, M and W polypeptides recognizing specific nucleic acid (DNA or RNA) sequence stretches can be generated from any of the following polypeptides:
- Modified (designed) TAL effectors (TALe),
- Modified (designed) zinc fingers (ZN),
- Relaxases, such as VirD2-like single strand DNA cleaving, and 5' end binding proteins, or TraI, TrwC and MobA. As an example, for MobA and its functional role as VirD2, see Bravo-Angel et al. (Bravo-Angel et al., 1999),
- VirE2-like modified single strand DNA binding proteins,
- RNA binding proteins, such as deaminases (Bass, 2002 and Iyer et al., 2011), P19 and similar viral dsRNA binding proteins (Silhavy et al., 2012).
- CRISPR associated protein 9 (Cas9) and the same family of proteins and their genetically and/or recombinantly engineered derivatives. These proteins have the ability to recognize short CRISPR derived RNAs (crRNAs), trans-activating-RNA (tracrRNA), guideRNAs and their hybrids with DNA or genetically and/or recombinantly engineered members recognizing DNA-DNA hybrids and cleave within the hybridized regions [as exemplified in (Jinek et al., 2012; Cong et al., 2013; Mali et al., 2013)],
- Transcription factors with known DNA binding specificity (e.g. WRKYs, NACs, DOFs, bZIP, MADS box and MYB transcription factors); and
- Other genetically and/or recombinantly engineered DNA binding proteins.

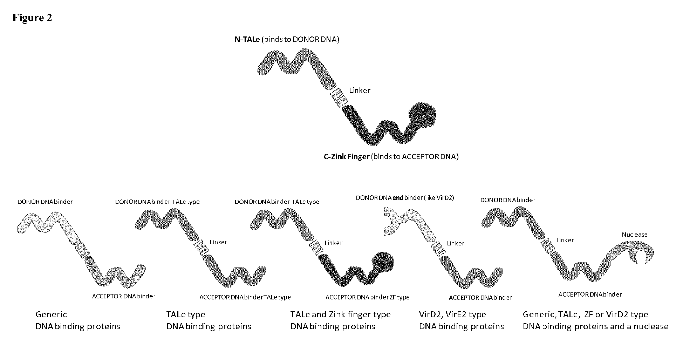

M an W peptides could be of the same type, but recognizing different stretches of nucleic acids, or different types, such as, for example, in cases where M is a TALe and W is a ZF (Figure 2).

Further preferred is a nucleic acid carrier molecule according to the present invention, wherein W is a fusion protein, for example comprising an enzyme, such as an endonuclease. Thus, the M or W molecule (polypeptide) could also be fused to enzymes to fulfill a function. As an example, the fusion of W to an endonuclease could be used to generate specific cuts in the target DNA upon binding of the MLW protein (Figure 2). As another example, in designing the MLW construct of the present invention, fused to TAL effector-encoding nucleic acid sequences can be are sequences encoding a nuclease or a portion of a nuclease, typically a nonspecific cleavage domain from a type II restriction endonucleases, such as FokI (Kim et al., 1996). Other useful endonucleases may include, for example, ISceI (Rouet et al., 1994), HhaI, HindIII, NotI, BbvCI, EcoRI, and BglI. The fact that some endonucleases (e.g., FokI) only function as dimers can be capitalized upon to enhance the target specificity of the TAL effector (Bitinaite et al., 1998). For example, in some cases each FokI monomer can be fused to a TAL effector sequence that recognizes a different DNA target sequence, and only when the two recognition sites are in close proximity do the inactive monomers come together to create a functional enzyme.

Even further preferred is a nucleic acid carrier molecule according to the present invention, wherein molecule comprises additional functional groups S₁ and/or S₂, wherein S₁ and S₂ are selected from the nucleus location signals (NLS), organelle targeting peptides (see, for example, Indio et al., 2013), and translocation signal polypeptides, such as, for example, type III or type IV (e.g. D2TS) translocation signal peptides. It was surprisingly found that the addition of a type IV translocation signal peptide (D2TS) to the C-terminus of an MLW construct (here; VirD2-L-TALE_{GFP}) significantly increased the expression of the expression of the nucleic acid (here: mCHERRY) bound to M (VirD2).

According to the present invention, any suitable type of nucleic acid can be introduced (and/or bound) by the nucleic acid carrier molecule according to the present invention. Preferably, the donor or target nucleic acid is selected from RNA, DNA, PNA and combinations thereof.

The nucleic acid carrier molecules according to the present invention can be used for gene targeting in cells and cells of tissues both *in vivo* and *in vitro.* Preferably, the nucleic acid carrier molecules according to the present invention are used in a cell selected from an animal or plant cell, such as, for example, a protoplast. Preferred is the use of the nucleic acid carrier molecules according to the present invention, wherein the organelle containing the target nucleic acid is selected from the group of a nucleus, a chloroplast and a mitochondrium.

The nucleic acid carrier molecule according to the present invention can be delivered by various means to cells and tissues to be transformed, i.e. host cells. These include:
- Cell penetrating peptides like TAT which have the ability to bind to DNA and protein complexes and facilitate their uptake to host cells. Similarly, such peptides could be translationally fused to the M and W polypeptides and eliminate the need for co-application;
- Various transfection methods, including PEG transformation;
- Microinjection;
- Silicon carbide whiskers;
- Ultrasound and shock waves;
- Electrophoresis;
- Laser microbeams;
- Microprojectile bombardment (e.g. gold particles);
- The use of *Agrobacterium* or *Bartonella* bacterial species that deliver DNA protein complexes using Type IV secretion systems. *Agrobacterium* is known to deliver such DNA protein complexes to fungi, plants, insect and even mammalian cells. *Bartonella* species were shown to transform human cell lines.
- MLW constructs could be delivered by bacterial species using Type III secretion system to infect eukaryotic cells followed by delivery of nucleic acids by standard techniques;
- MLW constructs could also be expressed under inducible promoters in transgenic plants and DNA can be delivered to these plants by *Agrobacterium*-mediated transformation or direct DNA delivery methods. After successful genome editing the MLW transgenic locus could be segregated out by backcrossing to wild type plants;
- *In vitro* expressed MLW molecules are introduced into the target cells, either as single molecules or bound to (or fused to) polypeptide carrier molecules as delivery vehicles.

The donor nucleic acid to be transformed can either be pre-bound to M and/or W of the MLW construct and delivered together to host cells and genomes, or introduced as naked nucleic acid by common DNA delivery methods to host cells transiently or stably expressing an MLW protein under regulated promoters.

Yet another aspect of the present invention then relates to a recombinant nucleic acid, encoding for a nucleic acid carrier molecule according to the present invention. Preferably, said recombinant nucleic acid is part of (is cloned into) an expression vector or an expression cassette, and is expressed, either transiently or permanently, or upon induction.

Yet another aspect of the present invention then relates to a method for recombinantly transforming a nucleic acid into an organelle in a cell, comprising the steps of a) providing a cell to be transformed comprising organelles, b) providing the nucleic acid carrier molecule according to the present invention in said organelles, c) providing a donor nucleic acid sequence in said organelles, and d) selecting cells comprising organelles, wherein said donor nucleic acid sequence has been recombinantly transformed into the DNA of said organelles. The method can be performed in vivo (e.g. in plants) and/or in vitro. Preferably, said cell is an animal or plant cell, such as, for example, a protoplast. More preferably, said organelle is selected from the group of a nucleus, a chloroplast and a mitochondrium.

Further preferably, said nucleic acid carrier molecule according to the present invention is provided as an expression vector according to the present invention, or an expression cassette according to the present invention.

As mentioned above, said method preferably comprises the use of a bacterium, such as, for example, *Agrobacterium tumefaciens, Sinorhizobium meliloti, Bartonella henselae, Helicobacter pylori, Pseudomonas aeruginosa, Pseudomonas syringae, Bacillus megaterium, E. coli* or other *Bartonella* bacterial species that could be engineered to deliver the constructs of the invention to animal cells. See also Kempf et al., 2002; da Cunha et al., 2007; Llosa et al., 2009; Schroeder et al., 2011; or Büttner, 2012

In a preferred embodiment, said transformation comprises gene targeting of a specific gene, such as a viral gene sequence, a mutated gene sequence, (for example point mutations, insertions, deletions and inversion of DNA sequences), transposon sequences or any other foreign (heterologous) sequence in a genome and a gene sequence that is organelle-specific, as described herein.

As mentioned above, the method comprises a transformation comprising bringing said first target nucleic acid sequence into close proximity with said second target nucleic acid sequence when both target nucleic acid sequences are bound to said carrier molecule.

Yet another aspect of the present invention then relates to the use of the nucleic acid carrier molecule according to the present invention, or the expression vector according to the present invention or the expression cassette according to the present invention for recombinantly transforming a nucleic acid in an organelle in a cell or group of cells *in vivo* or *in vitro,* as described herein. Preferred is a use, wherein said cell is an animal or plant cell, such as, for example, a protoplast. Further preferred is a use wherein said organelle is selected from the group of a nucleus, a chloroplast and a mitochondrium. However, the technology is not limited to engineering genomes and can be used in any synthetic biology applications and development of methods, tools and kits allowing targeted nucleic acid matching (bringing together) in complex environments including signaling networks in development of future (biological) computer chips.

Yet another aspect of the present invention then relates to a method for treating a disease in a subject, comprising recombinantly transforming a nucleic acid into an organelle in a cell of said subject, comprising the steps of comprising the steps of a) providing a cell to be transformed comprising organelles, b) providing the nucleic acid carrier molecule according to the present invention in said organelles, c) providing a donor nucleic acid sequence in said organelles, and d) selecting cells comprising organelles, wherein said donor nucleic acid sequence has been recombinantly transformed into the DNA of said organelles, wherein said donor nucleic acid sequence provides genetic information to the target nucleic acid that is effective in treating said disease. The method can be performed *in vivo* (e.g. in plants) and/or *in vitro.*

Examples for treatments are viral sequences integrating into host genomes, like the HIV virus or other integrating human, animal or plant viruses, which can be permanently deactivated by insertion or deletion of donor DNA sequences, and viral host receptors can be mutated to block virus infections. Human, animal and plant diseases and disorders linked to genetic defects can also be efficiently repaired using MLW proteins, if the underlying genetic origin is known. Other preferred aspects of the method include the precise delivery of miRNA, RNAi, antisense RNAs, crRNAs and guide RNAs to desired locations in genomes for efficient gene therapy in humans, animals and plants, the delivery of single strand or double strand oligos to desired locations in genomes for genome editing, and stem cell genome editing and subsequent correction of faulty alleles of human, animal or plant genes causing abnormalities and diseases.

Preferably, said cell to be treated is an animal or plant cell, such as, for example, a protoplast. More preferably, said organelle is selected from the group of a nucleus, a chloroplast and a mitochondrium.

Further preferably, said nucleic acid carrier molecule according to the present invention is provided as an expression vector according to the present invention, or an expression cassette according to the present invention.

The method preferably comprises the use of a bacterium, such as, for example, *Agrobacterium tumefaciens, Sinorhizobium meliloti, Bartonella henselae, Helicobacter pylori, Pseudomonas aeruginosa, Pseudomonas syringae, E. coli* or other *Bartonella* bacterial species.

In a preferred embodiment, the transformation comprises gene targeting of a specific gene, such as a viral gene sequence, a mutated gene sequence, and a gene sequence that is organelle-specific, as described herein.

The present invention will now be described further in the following examples, nevertheless, without being limited thereto. For the purposes of the present invention, all references as cited herein are incorporated by reference in their entireties. The Figures show:
Figure 1: A schematic overview of an MLW construct according to the present invention.
Figure 2: A schematic overview of several MLW constructs according to the present invention. TALE indicates TAL-effector elements (which in each case are designed for specific sequences, and can be the same or different), ZNF indicates a Zinc finger domain, and DNA binder indicates a nucleic acid binder (shown with bound nucleic acid), e.g. Vir2D. Nuclease indicates a nuclease domain, e.g., an endonucleases. δ indicates the close proximity distance (as explained above). MLW proteins can be derived from the same or different type of nucleic acid binding proteins.
Figure 3: A schematic overview of an MLW construct according to the present invention indicating the distances for the proximities as desired/required. The MLW proteins bring donor nucleic acids in very close distance (proximity) to target nucleic acids.

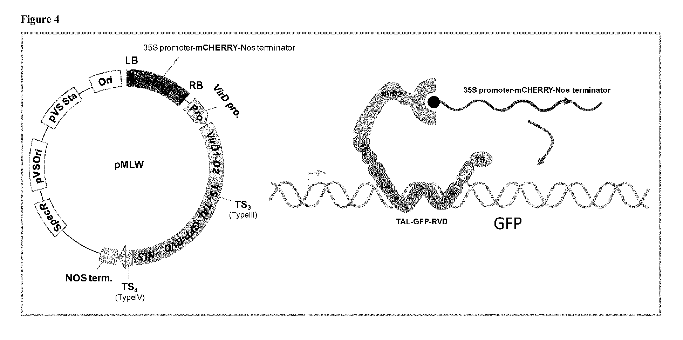

Figure 4: The vector map of the MLW construct VirD2-TALE_{GFP}, its protein product bound to T-DNA containing 35S promoter-mCHERRY-Nos terminator, and predicted targeting and binding to the GFP coding sequence in the plant genome.

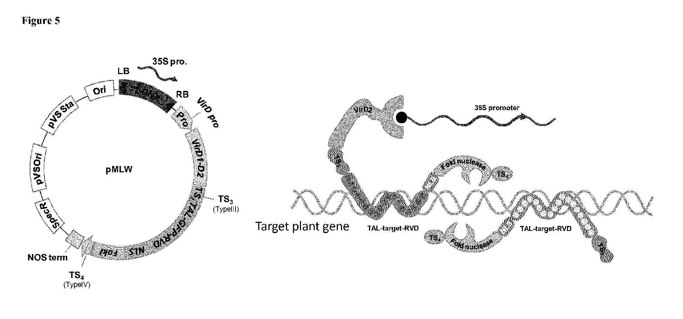

Figure 5: The vector map of the MLW construct VirD2-TALE_{target} plant gene, its protein product bound to T-DNA containing 35S promoter, and predicted targeting and binding to the targeted plant gene coding sequence in order to generate DNA cleavage and insert the VirD2 bound constitutively active 35S promoter. The expression of this plant gene is expected to increase upon insertion of 35S promoter. Similarly, any other sequence brought by the MLW protein is expected to be integrated into the FokI cleavage site in the genome.

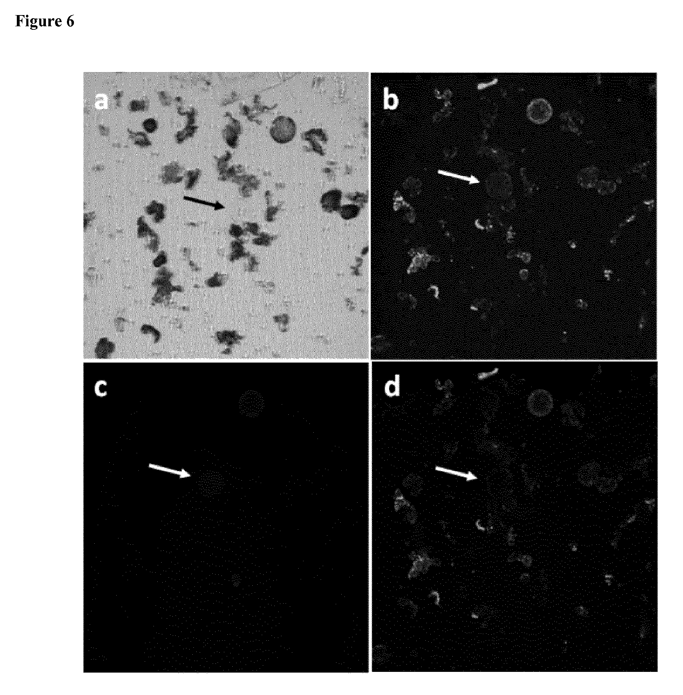

Figure 6: Photos of gene targeting events as performed with the MLW constructs of the present invention. The images shown in this figure are from protoplasts isolated 9 days after *Agrobacterium* infiltration. Plants were infiltrated with *Agrobacterium tumefaciens virD2* deletion strain carrying a binary vector containing the mCHERRY expression cassette within the T-DNA (between LB and RB) and the MLW construct VirD2-TALEGFP-D2TS outside of the T-DNA. To determine the expression of GFP and mCHERRY, different filters were used to image the same cells. The arrow indicates the protoplast where gene targeting event likely took place. (a) bright field image, (b) Merged image (RFP and GFP) of a gene targeting event, (c) image obtained using only RFP filter, (d) image obtained using only GFP filter. As seen in this figure, a GFP expressing cell has been targeted with mCHERRY construct since we can no longer detect GFP expression from this cell (see image d obtained using GFP filter allowing the detection of only GFP but not mCHERRY). However, the same cell is expressing strongly the mCHERRY (see c) obtained using RFP filter allowing the detection of only mCHERRY but not GFP).

### Examples

The following experiments have been performed using MLW constructs of the present invention in plant cells. Nevertheless, the person of skill will be able to readily transfer the principles of these experiments to other cells and tissues, such as bacteria or animal and human cells.

### Example 1

### Construction of MLW constructs

The following MLW molecules were designed.

**Table 1:**

| **No.** | **M** | **S₁** | **L** | **W** | **S₂** | **Nuclease (FokI)** |
|---|---|---|---|---|---|---|
| 1 | VirD2 | - | no | TALE_{GFP} (targeting GFP sequence in a transgenic plant containing GFP coding sequence) | - | - |
| 1a | VirD2 | - | no | TALE_{GFP} | D2TS (Type IV translocation signal from VirD2) | - |
| 2 | VirD2 | - | no | TALE_{GFP} | - | + |
| 3 | TALE_{mCHERRY} A TAL effector binding to mCHERRY | | yes | TALE_{GFP} A TAL effector binding to GFP | | - |
| 4 | TALE_{mCHERRY} | | yes | TALE_{GFP} | | + |
| 5 | VirD2 | | yes | TALE_{Endo} (targeting a plant endogenous gene) | | - |
| 6 | TALE_{mCHERRY} | | yes | TALE_{Endo} (targeting a plant endogenous gene) | | + |
| 7 | VirD2 | - | yes | TALE_{Endo-sense} (targeting a plant endogenous gene) | D2TS | + |
| 7b | - | - | - | TALE_{Endo-antisense} (targeting a plant endogenous gene) | D2TS | + |

VirD2 - VirD2 protein of *Agrobacterium*; TALE_{mCHERRY} - TAL-effector specific for mCHERRY; TALE_{GFP} TAL-effector specific for GFP; The construct VirD2-TALE_{GFP} was furthermore generated with a Type IV translocation signal (D2TS).

In transient transformation assays using virD2 mutant *Agrobacterium tumefaciens* strain D2-(Bravo-Angel et al., 1998), the inventors were able to demonstrate that the fusion constructs of VirD2-TALE_{GFP} complemented the mutation for the virD2 gene in this strain, since the mCHERRY expression cassette located on a T-DNA plasmid was successfully transferred to plant cells and lead to the expression of mCHERRY (see Table 2). The VirD2 *Agrobacterium tumefaciens* mutant strain could not transform plant cells if the inventors just supplied the mCHERRY expression cassette on a T-DNA, indicating that the strain is indeed virD2 mutant (see Table 2). Interestingly, it was found that the addition of a type IV translocation signal increased the mCHERRY expression significantly than the construct without the translocation signal.

**Table 2: Results using the MLW construct VirD2-TALE_{GFP}**

| *Agrobacterium* strain | Expression cassette | MLW construct | Transient transformation assay (mCHERRY) | Targeting GFP |
|---|---|---|---|---|
| *A. tumefaciens virD2* mutant | 35S pro-mCHERRY-Nos term | - | No expression detected | - |
| *A. tumefaciens virD2* mutant | 35S pro-mCHERRY-Nos term | VirD2-TALE_{GFP} | + | Not tested |
| *A. tumefaciens virD2* mutant | 35S pro-mCHERRY-Nos term | VirD2-TALE_{GFP} D2TS | ++ | + |
| *A. tumefaciens* GV3101 pMP90, wildtype *virD2* | 35S pro-mCHERRY-Nos term | - | +++ | - |

The inventors have performed experiments to determine whether the GFP locus in a transgenic tobacco plant containing GFP in a heterozygous state can be targeted using the first MLW constructs. For the first experiment, the inventors chose to perform the Agrobacterium infiltration and transient transformation assays just 36 hours after infiltration of bacteria. Protoplasts were isolated from transgenic GFP positive tobacco plant leaves infiltrated with bacterium containing constructs.

Using different filters, images of the same cells were obtained to determine the expression of GFP and mCHERRY. The images in Figure 3 were taken from protoplasts isolated from transgenic GFP positive tobacco plant leaves infiltrated with *Agrobacterium* containing the targeting constructs. A GFP expressing cell was targeted with an mCHERRY construct since GFP expression from this cell can no longer be detected (see image c obtained using GFP filter allowing the detection of only GFP, but not mCHERRY). However, the same cell strongly expresses mCHERRY (see image b obtained using RFP filter allows the detection of only mCHERRY but not GFP). This is a very strong indication of gene targeting. From this preliminary experiment, it could be shown that the fusion construct to VirD2 is functional and can complement the virD2 mutant *Agrobacterium.* Furthermore, the first preliminary evidence of gene targeting using the construct could be obtained. As expected, the control transformation constructs did not produce any targeting.

### Example 2

This example relates to an MLW protein containing VirD2 and a designed TAL-effector (MLW-D2TALe) and its use in targeted genome insertions in plants (Figure 4a).

To demonstrate that the inventors can target a locus in plants and insert a transgene there, GFP expressing transgenic Arabidopsis plants are used, and the expression of the GFP gene is eliminated and it is converted it into mCherry expression by the inserted T-DNA. To achieve this the inventors engineer a TAL-effector that would specifically bind to a region in the GFP coding sequence. This effector will be translationally fused to VirD2 relaxase in *Agrobacterium.* If the VirD2-TALe-GFP fusion protein (MLW protein) is functional, it will lead to T-DNA processing, binding to RB and taking the T-DNA strand through the type IV channel into the plant cells and nucleus (Figure 4a). In the nucleus, the selective sequence binding activity of TALe-GFP will bring the whole complex to the GFP locus. This in turn should increase the probability of T-DNA insertion into the GFP by several magnitudes. The T-DNA sequence that is delivered with VirD2-TALe-GFP protein will contain the coding sequence of the mCherry under the control of a constitutive promoter. The mCherry coding sequence, due to its very high homology to GFP, will also help for homologous recombination based insertion.

Finally, the insertion of mCherry coding sequence into GFP coding sequence will lead to a loss of GFP expression and presence of mCherry expression. This expected approach can be extended to native plant genes and methods for their manipulations, as well as to targeted deletions of plant sequences.

One of the most important advantage of the use of VirD2 as M polypeptide is that any DNA cloned into a vector containing the cleavage sites RB and LB can be cleaved by this protein and transferred to host cells as covalent-bound VirD2-DNA strand. The W protein linked to this complex should translocate it to the acceptor DNA region in the genome. This eliminates the need to design any specific M polypeptides containing designed TALes or ZFs. Similarly, once a functional W protein found effectively bringing a DNA/protein complex to a defined acceptor sequence in genome, any other DNA sequences could be introduced in this locus without any need for designing an M domain or polypeptide.

### Example 3

This example relates to an MLW protein containing two different TAL effector binding sites for targeted insertion and expression of a transgene in plants. (Figure 4b).

The goal of this experiment is similar to Example 3, but the MLW protein and DNA/protein complex delivery method is different. With wanting to be bound by theory, the inventors speculate that the MLW protein containing VirD2-TALe-GFP may be too large to be translocated by the Type IV secretion system of *Agrobacterium.* The use of a direct DNA/protein complex delivery method should circumvent such a potential problem. In this experiment two designed TAL effectors will be used in the MLW protein for genome editing in plants (Figure 4b), In this experiment, the inventors will also use the MLW protein VirD2-TALe-GFP generated in Example 1. This protein will be expressed *in E coli.* Using the purified protein mixed with a vector containing mCherry transgene flanked by VirD2 cleavage sites RB and LB, it is expected that majority of the MLW protein is bound to the 5' end of the cleaved DNA, due to the presence of VirD2 in the MLW. This DNA/protein complex will then be delivered to plant cells using various direct delivery methods as described above.

### Example 4

This example relates to targeting a GFP locus in a transgenic tobacco plant containing GFP in a heterozygous state (Figure 6).

In order to demonstrate gene targeting using MLW proteins, the inventors targeted a GFP locus in a transgenic tobacco plant containing GFP in a heterozygous state. This allowed a single targeting event to sufficient for eliminating GFP expression. Assaying for gene targeting was performed 36 hours or 9 days after *Agrobacterium* infiltration mediated transient transformation assay. Since the plant cell wall components appear to be re-arranged by the plant and the invading *Agrobacterium* in such infection assays, the protoplasting efficiency and quality of the preparation were low from *Agrobacterium* infilatrated plant tissues 9 days after infiltration. Very good protoplasting efficiency and purity from leaves collected 36-hours post infiltration were obtained (data not shown).

However, since this is a rather early time point for gene targeting and DNA repair to take place, no positive events were detected from such material. Therefore, the images shown in Figure 6 are from protoplasts isolated 9 days after *Agrobacterium* infiltration. Plants were infiltrated with *Agrobacterium tumefaciens virD2* deletion strain carrying a binary vector containing the mCHERRY expression cassette within the T-DNA (between LB and RB) and the MLW construct VirD2-TALEGFP-D2TS outside of the T-DNA. To determine the expression of GFP and mCHERRY, different filters were used to image the same cells. The arrow indicates the protoplast where gene targeting event likely took place. (a) bright field image, (b) Merged image (RFP and GFP) of a gene targeting event, (c) image obtained using only RFP filter, (d) image obtained using only GFP filter. As seen in this figure, a GFP expressing cell has been targeted with mCHERRY construct since we can no longer detect GFP expression from this cell (see image d obtained using GFP filter allowing the detection of only GFP but not mCHERRY). However, the same cell is strongly expressing the mCHERRY (see Figure 6 c) obtained using RFP filter allowing the detection of only mCHERRY but not GFP).

### References as cited:

Alonso, J.M., et al. (2003). Genome-wide insertional mutagenesis of Arabidopsis thaliana. Science 301: 653-657.
Bass, B. L. (2002). "RNA editing by adenosine deaminases that act on RNA." Annual Review of Biochemistry 71(1): 817-846.
Bitinaite, J., Wah, D.A., Aggarwal, A.K., and Schildkraut, I. (1998). FokI dimerization is required for DNA cleavage. Proc Natl Acad Sci U S A 95: 10570-10575.
Boch, J., et al. (2009). Breaking the code of DNA binding specificity of TAL-type III effectors. Science 326: 1509-1512.
Bravo-Angel, A.M., Hohn, B., and Tinland, B. (1998). The omega sequence of VirD2 is important but not essential for efficient transfer of T-DNA by Agrobacterium tumefaciens. Mol Plant Microbe Interact 11: 57-63.
Bravo-Angel, A.M., Gloeckler, V., Hohn, B., and Tinland, B. (1999). Bacterial conjugation protein MobA mediates integration of complex DNA structures into plant cells. J Bacteriol 181: 5758-5765.
Büttner, D. (2012). Protein Export According to Schedule: Architecture, Assembly, and Regulation of Type III Secretion Systems from Plant- and Animal-Pathogenic Bacteria. Microbiology and Molecular Biology Reviews 76: 262-310.
Cai, C., et al. (2009). Targeted transgene integration in plant cells using designed zinc finger nucleases. Plant Molecular Biology 69: 699-709.
Cermak, T., Doyle, E.L., Christian, M., Wang, L., Zhang, Y., Schmidt, C., Baller, J.A., Somia, N.V., Bogdanove, A.J., and Voytas, D.F. (2011). Efficient design and assembly of custom TALEN and other TAL effector-based constructs for DNA targeting. Nucleic Acids Res 39: e82.
Citovsky, V., Zaltsman, A., Kozlovsky, S.V., Gafni, Y., and Krichevsky, A. (2009). Proteasomal degradation in plant-pathogen interactions. Semin Cell Dev Biol.
Cong, L., et al. (2013). Multiplex Genome Engineering Using CRISPR/Cas Systems. Science 339: 819-823.
da Cunha, L., Sreerekha, M.V., and Mackey, D. (2007). Defense suppression by virulence effectors of bacterial phytopathogens. Curr Opin Plant Biol 10: 349-357.
Durrenberger, F., Crameri, A., Hohn, B., and Koukolikova-Nicola, Z. (1989). Covalently bound VirD2 protein of Agrobacterium tumefaciens protects the T-DNA from exonucleolytic degradation. Proc Natl Acad Sci U S A 86: 9154-9158.
Fauser, F., Roth, N., Pacher, M., Ilg, G., Sanchez-Fernandez, R., Biesgen, C., and Puchta, H. (2012). In planta gene targeting. Proc Natl Acad Sci U S A 109: 7535-7540.
Gelvin, S.B. (2009). Agrobacterium in the genomics age. Plant Physiol 150: 1665-1676.
Indio V, Martelli PL, Savojardo C, Fariselli P, Casadio R. The prediction of organelle-targeting peptides in eukaryotic proteins with Grammatical-Restrained Hidden Conditional Random Fields.Bioinformatics. 2013 Apr 15;29(8):981-8.
Iyer, L. M., D. Zhang, et al. (2011). "Evolution of the deaminase fold and multiple origins of eukaryotic editing and mutagenic nucleic acid deaminases from bacterial toxin systems." Nucleic Acids Res 39(22): 9473-97.
Jarchow, E., Grimsley, N.H., and Hohn, B. (1991). virF, the host-range-determining virulence gene of Agrobacterium tumefaciens, affects T-DNA transfer to Zea mays. Proc Natl Acad Sci U S A 88: 10426-10430.
Jayaswal, R.K., Veluthambi, K., Gelvin, S.B., and Slightom, J.L. (1987). Double-stranded cleavage of T-DNA and generation of single-stranded T-DNA molecules in Escherichia coli by a virD-encoded border-specific endonuclease from Agrobacterium tumefaciens. J Bacteriol 169: 5035-5045.
Jinek, M., Chylinski, K., Fonfara, I., Hauer, M., Doudna, J.A., and Charpentier, E. (2012). A programmable dual-RNA-guided DNA endonuclease in adaptive bacterial immunity. Science 337: 816-821.
Kay, S., and Bonas, U. (2009). How Xanthomonas type III effectors manipulate the host plant. Curr Opin Microbiol 12: 37-43.
Kay, S., Hahn, S., Marois, E., Hause, G., and Bonas, U. (2007). A Bacterial Effector Acts as a Plant Transcription Factor and Induces a Cell Size Regulator. Science 318: 648-651.
Kempf, V.A., Hitziger, N., Riess, T., and Autenrieth, I.B. (2002). Do plant and human pathogens have a common pathogenicity strategy? Trends Microbiol 10: 269-275.
Kim, Y.G., Cha, J., and Chandrasegaran, S. (1996). Hybrid restriction enzymes: zinc finger fusions to Fok I cleavage domain. Proc Natl Acad Sci U S A 93: 1156-1160.
Lacroix, B., Tzfira, T., Vainstein, A., and Citovsky, V. (2006). A case of promiscuity: Agrobacterium's endless hunt for new partners. Trends Genet 22: 29-37.
Lessl, M., and Lanka, E. (1994). Common mechanisms in bacterial conjugation and Ti-mediated T-DNA transfer to plant cells. Cell 77: 321-324.
Li, T., Liu, B., Spalding, M.H., Weeks, D.P., and Yang, B. (2012). High-efficiency TALEN-based gene editing produces disease-resistant rice. Nat Biotechnol 30: 390-392.
Llosa, M., Roy, C., and Dehio, C. (2009). Bacterial type IV secretion systems in human disease. Mol Microbiol 73: 141-151.
Lloyd, A., Plaisier, C.L., Carroll, D., and Drews, G.N. (2005). Targeted mutagenesis using zinc-finger nucleases in Arabidopsis. Proc Natl Acad Sci U S A 102: 2232-2237.
Magori, S., and Citovsky, V. (2012). The role of the ubiquitin-proteasome system in Agrobacterium tumefaciens-mediated genetic transformation of plants. Plant Physiol 160: 65-71.
Mahfouz, M.M., Li, L., Piatek, M., Fang, X., Mansour, H., Bangarusamy, D.K., and Zhu, J.K. (2012). Targeted transcriptional repression using a chimeric TALE-SRDX repressor protein. Plant Mol Biol 78: 311-321.
Mali, P., Yang, L., Esvelt, K.M., Aach, J., Guell, M., DiCarlo, J.E., Norville, J.E., and Church, G.M. (2013). RNA-Guided Human Genome Engineering via Cas9. Science.
McCullen, C.A., and Binns, A.N. (2006). Agrobacterium tumefaciens and plant cell interactions and activities required for interkingdom macromolecular transfer. Annu Rev Cell Dev Biol 22: 101-127.
Miller, J.C., et al. (2011). A TALE nuclease architecture for efficient genome editing. Nat Biotechnol 29: 143-148.
Morbitzer, R., Romer, P., Boch, J., and Lahaye, T. (2010). Regulation of selected genome loci using de novo-engineered transcription activator-like effector (TALE)-type transcription factors. Proc Natl Acad Sci U S A 107: 21617-21622.
Moscou, M.J., and Bogdanove, A.J. (2009). A simple cipher governs DNA recognition by TAL effectors. Science 326: 1501.
Pitzschke, A., and Hirt, H. (2010). New insights into an old story: Agrobacterium-induced tumour formation in plants by plant transformation. EMBO J 29: 1021-1032.
Romer, P., Recht, S., Strauss, T., Elsaesser, J., Schornack, S., Boch, J., Wang, S., and Lahaye, T. (2010). Promoter elements of rice susceptibility genes are bound and activated by specific TAL effectors from the bacterial blight pathogen, Xanthomonas oryzae pv. oryzae. New Phytol 187: 1048-1057.
Rouet, P., Smih, F., and Jasin, M. (1994). Expression of a site-specific endonuclease stimulates homologous recombination in mammalian cells. Proc Natl Acad Sci U S A 91: 6064-6068.
Scheiffele, P., Pansegrau, W., and Lanka, E. (1995). Initiation of Agrobacterium tumefaciens T-DNA processing. Purified proteins VirD1 and VirD2 catalyze site- and strand-specific cleavage of superhelical T-border DNA in vitro. J Biol Chem 270: 1269-1276.
Schroeder, G., Schuelein, R., Quebatte, M., and Dehio, C. (2011). Conjugative DNA transfer into human cells by the VirB/VirD4 type IV secretion system of the bacterial pathogen Bartonella henselae. Proceedings of the National Academy of Sciences.
Shukla, V.K., et al. (2009). Precise genome modification in the crop species Zea mays using zinc-finger nucleases. Nature 459: 437-441.
Silhavy, D., A. Molnar, et al. (2002). "A viral protein suppresses RNA silencing and binds silencing-generated, 21- to 25-nucleotide double-stranded RNAs." EMBO J 21(12): 3070-80.
Townsend, J.A., Wright, D.A., Winfrey, R.J., Fu, F., Maeder, M.L., Joung, J.K., and Voytas, D.F. (2009). High-frequency modification of plant genes using engineered zinc-finger nucleases. Nature 459: 442-445.
Tzfira, T., and Citovsky, V. (2006). Agrobacterium-mediated genetic transformation of plants: biology and biotechnology. Curr Opin Biotechnol 17: 147-154.
Tzfira, T., Vaidya, M., and Citovsky, V. (2004). Involvement of targeted proteolysis in plant genetic transformation by Agrobacterium. Nature 431: 87-92.
Ülker, B., Allen, G.C., Thompson, W.F., Spiker, S., and Weissinger, A.K. (1999). A tobacco matrix attachment region reduces the loss of transgene expression in the progeny of transgenic tobacco plants. The Plant Journal 18: 253-263.
Urnov, F.D., Rebar, E.J., Holmes, M.C., Zhang, H.S., and Gregory, P.D. (2010). Genome editing with engineered zinc finger nucleases. Nat Rev Genet 11: 636-646.
Vogel, A.M., and Das, A. (1992). Mutational analysis of Agrobacterium tumefaciens virD2: tyrosine 29 is essential for endonuclease activity. J Bacteriol 174: 303-308.
Wright, D.A., Townsend, J.A., Winfrey, R.J., Jr., Irwin, P.A., Rajagopal, J., Lonosky, P.M., Hall, B.D., Jondle, M.D., and Voytas, D.F. (2005). High-frequency homologous recombination in plants mediated by zinc-finger nucleases. Plant J 44: 693-705.
Ziemienowicz, A., Merkle, T., Schoumacher, F., Hohn, B., and Rossi, L. (2001). Import of Agrobacterium T-DNA into plant nuclei: two distinct functions of VirD2 and VirE2 proteins. Plant Cell 13: 369-383.

## Claims

1. A nucleic acid carrier molecule, comprising the general formula
M-S₁-L-W-S₂,
wherein M is a first polypeptide specifically binding to a donor nucleic acid sequence to be transferred into an organelle of a cell,
W is a second polypeptide specifically binding to a target nucleic acid sequence, wherein said target nucleic acid sequence is located in an organelle of a cell,
L is missing or is linking group allowing M and W flexibility and semi-independence, and
S₁ and S₂ independently of each other are missing or are a signal peptide sequence, wherein said donor nucleic acid sequence is brought into close proximity with said target nucleic acid sequence when both nucleic acid sequences are bound to said carrier molecule.

2. The nucleic acid carrier molecule according to claim 1, wherein M is selected from the group of a TAL effector (TALe) polypeptide, a zinc finger polypeptide, a relaxase, such as, for example, VirD2-like polypeptide, a VirE2-like polypeptide, an RNA binding polypeptide, CRISPR associated protein 9 (Cas9) family proteins and their derivatives, and a transcription factor polypeptide, wherein said polypeptide specifically binds to said donor nucleic acid, and preferably is a fusion protein, for example comprising an enzyme, such as an endonuclease.

3. The nucleic acid carrier molecule according to claim 1 or 2, wherein W is selected from the group of a TAL effector (TALe) polypeptide, a zinc finger polypeptide, a VirD2-like polypeptide, a VirE2-like polypeptide, an RNA binding polypeptide, and a transcription factor polypeptide, wherein said polypeptide specifically binds to said target nucleic acid sequence that is located in said organelle, and wherein W preferably is a fusion protein, for example comprising an enzyme, such as an endonuclease.

4. The nucleic acid carrier molecule according to any of claims 1 to 3, wherein L is selected from a polypeptide linker or an organic linker group which is covalently connecting M and W.

5. The nucleic acid carrier molecule according to any of claims 1 to 4, wherein S₁ and S₂ are selected from the group of translocation signal polypeptides, such as type IV translocation signal (D2TS) peptides, organelle targeting peptides, and type III translocation signals.

6. The nucleic acid carrier molecule according to any of claims 1 to 5, wherein said target nucleic acid sequence is selected from viral sequences, mutated sequences, transposon sequences or any other foreign sequence in a genome and sequences that are organelle-specific.

7. The nucleic acid carrier molecule according to any of claims 1 to 6, wherein said cell is an animal or plant cell, such as, for example, a protoplast.

8. The nucleic acid carrier molecule according to any of claims 1 to 7, wherein said organelle is selected from the group of a nucleus, a chloroplast and a mitochondrium.

9. A recombinant nucleic acid, encoding for a nucleic acid carrier molecule according to any of claims 1 to 8, which is preferably part of an expression vector or an expression cassette.

10. An *in vitro* method for recombinantly transforming a nucleic acid into an organelle in a cell, comprising the steps of
a) providing a cell to be transformed comprising organelles,
b) providing the nucleic acid carrier molecule according to any of claims 1 to 8 in said organelles,
c) providing a donor nucleic acid sequence in said organelles, and
d) selecting cells comprising organelles wherein said donor nucleic acid sequence has been recombinantly transformed into the DNA of said organelles.

11. The method according to claim 10, wherein said cell is an animal or plant cell, such as, for example, a protoplast, or a stem cell, wherein human embryonic stem cells are excluded, and wherein said organelle preferably is selected from the group of a nucleus, a chloroplast and a mitochondrium.

12. The method according to any of claims 10 or 11, wherein said method comprises the use of a bacterium, such as, for example, *Agrobacterium tumefaciens, Sinorhizobium meliloti, Bartonella henselae, Helicobacter pylori, Pseudomonas aeruginosa, Pseudomonas syringae, Bacillus megaterium,* and *E. coli.*

13. The method according to any of claims 10 to 12, wherein said transformation comprises gene targeting of a specific gene, such as a viral gene sequence, a mutated gene sequence, and a gene sequence that is organelle-specific.

14. The method according to any of claims 10 to 13, wherein said transformation comprises bringing said first target nucleic acid sequence into close proximity with said second target nucleic acid sequence when both target nucleic acid sequences are bound to said carrier molecule.

15. Use of the nucleic acid carrier molecule according to any of claims 1 to 8, or the expression vector or the expression cassette according to claim 9 for recombinantly transforming a nucleic acid in an organelle in a cell *in vitro* or group of cells *in vivo,* wherein said cell preferably is an animal or plant cell, such as, for example, a protoplast, and wherein said organelle preferably is selected from the group of a nucleus, a chloroplast, and a mitochondrium.
